# EUROPEAN PATENT SPECIFICATION

(11) **EP 2 874 586 B1**
(45) Date of publication and mention of the grant of the patent: **10.01.2018**
(21) Application number: 13715482.9
(22) Date of filing: 12.03.2013
(51) Int. Cl.: A61F 13/00, A61F 13/02, A61F 13/58, A61F 15/00

(54) **A SECUREMENT ASSEMBLY**
SICHERUNGSANORDNUNG
ENSEMBLE D'IMMOBILISATION

(30) Priority: 20.07.2012 US 201261673868 P
(43) Date of publication of application: 27.05.2015
(73) Proprietor: 3M Innovative Properties Company, St. Paul, MN 55133-3427 (US)
(72) Inventor: LIU, Junkang J., Saint Paul, Minnesota 55133-3427 (US); DIETZ, Timothy M., Saint Paul, Minnesota 55133-3427 (US); WOOD, Leigh E., Saint Paul, Minnesota 55133-3427 (US); GORMAN, Michael R., Saint Paul, Minnesota 55133-3427 (US); KARIM, Naimul, Saint Paul, Minnesota 55133-3427 (US); SANGHI, Shilpi K., Saint Paul, Minnesota 55133-3427 (US)
(74) Representative: Vossius & Partner Patentanwälte Rechtsanwälte mbB
(86) International application number: PCT/US2013/030431
(87) International publication number: WO 2014/014504

(56) References cited:
- EP-A1- 0 321 234
- WO-A1-2004/058122
- US-A1- 2010 307 668
- US-B1- 6 195 850
- US-B1- 6 545 193

## Description

### Field

The present disclosure relates to a securement assembly comprising an adhering element and a securing element.

### Background

Adhesive tapes and medical dressings are used in a variety of application for securing material to a substrate. In some applications, the adhesive of the tape may secure well to the substrate, but not be well suited for securing with the device. For example, silicone adhesives do not strongly secure to polymeric devices. In some applications, placement of the device directly in contact with the substrate, over which the adhesive tape is applied, can cause damage to the underlying substrate. For example, rigid devices placed adjacent to skin for an extended period of time can cause skin irritation or even pressure ulcers.

WO 2004/058122 discloses a medical wrap which includes a flexible, sheet-form substrate with a discrete medical function delivery region having a surface adapted to be placed against a patient's skin to provide a desired medical effect.

US 6,545,193 discloses an elongated elastic bandage which includes a plurality of hook fastener strips on a first side and a plurality of loop fastener strips on a second side.

US 2010/307668 relates to a method for folding a fastener during a high speed manufacturing process and maintaining the fastener in a folded configuration throughout the high speed manufacturing process.

EP 321 234 discloses a disposable absorbent article with a mechanical fastening system having disposal means so as to provide convenient disposal of the absorbent article.

US 6,195,850 discloses a closure system and method of making a closure system for a disposable article. One face of a continuous closure system web is provided with first fastening region at terminal end portion(s) of the web.

### Summary

The present invention is defined by the features of independent claim 1. Further preferred embodiments of the invention are defined in the dependent claims. In particular,the securement assembly uses a combination of adhesive as well as mechanical fasteners for effectively securing a device to a skin surface. The securement assembly can be used to protect the skin surface from the device being secured. The securement assembly comprises an adhering element with a first mechanical fastener and a securing element with a second mechanical fastener, wherein in some embodiments the securing element can serve as both a cover for the adhesive on the adhering element while also providing the securement between the first mechanical fastener and second mechanical fastener.

In one embodiment, the securement assembly comprises an adhering element and a securing element. The adhering element comprises a first surface comprising a first mechanical fastener, a second surface, opposite the first surface, comprising an adhesive. The securing element comprises a first surface, a second surface, opposite the first surface, a second mechanical fastener on at least the first surface or second surface of the securing element, wherein the second mechanical fastener is configured to secure with the first mechanical fastener. The first surface of the securing element covers and releasably secures to the adhesive of the second surface of the adhering element.

In one embodiment the securement assembly comprises and adhering element and a securing element. The adhering element comprises a first surface comprising a first mechanical fastener, a second surface, opposite the first surface, comprising an adhesive. The securing element, removable from the adhering element, comprises a first surface, a second surface, opposite the first surface, a second mechanical fastener on at least one of the first surface or second surface of the securing element. The second mechanical fastener secures to the first mechanical fastener.

In one embodiment, the securement assembly comprises an adhering element and a securing element. The adhering element comprises a first surface comprising a first plurality of stems or hooks, a second surface, opposite the first surface, comprising an adhesive. The securing element comprises a first surface, a second surface, opposite the first surface, comprising a second plurality of stems or hooks. A working adhesive is partially embedded into at least one of the first or second plurality of stems or hooks. The first plurality of stems or hooks secures to the second plurality of stems or hooks. A device is positioned between a portion of the first surface of the adhering element and the securing element.

In one embodiment, (not part of the invention) a method of securing a tape comprises providing a securement assembly, wherein the securement assembly comprises an adhering element comprising a first surface comprising a first mechanical fastener and a second surface, opposite the first surface, comprising an adhesive, a securing element comprising a first surface and a second surface, opposite the first surface, and a second mechanical fastener on at least one of the first surface or second surface of the securing element to secure with the first mechanical fastener, wherein the securing element covers the adhesive of the second surface of the adhering tape, and removing the securing element from the adhering element, and securing the adhesive of the second surface of the adhering element to a surface.

In one embodiment, (not part of the invention) a method of securing a device comprises providing an adhering element comprising a first surface comprising a first plurality of stems or hooks and a second surface, opposite the first surface, comprising an adhesive, securing the adhesive to a surface, providing a securing element comprising a first surface and a second surface, opposite the first surface, comprising a second plurality of stems or hooks, placing a device on the adhering element, covering at least a portion of the device with the securing element, securing the first plurality of stems or hooks with the second plurality of stems or hooks, where a working adhesive is partially embedded into at least one of the first or second plurality of stems or hooks.

### Brief Description of the Drawings

FIG. 1 is a side sectional view of a first embodiment of a securement assembly;
FIG. 2 is a side sectional view of a second embodiment of a securement assembly;
FIG. 3 is a side sectional view of the adhering element of FIG. 1;
FIG. 4 is a top view of the adhering element of FIG. 3;
FIG. 5 is a side sectional view of the securing element of FIG. 1 or 2;
FIG. 6 is a bottom view of the securing element of FIG. 5;
FIG. 7 is a side sectional view of the adhering element shown securing a device to a surface;
FIG. 8 is a side sectional view of the adhering element and securing element shown securing a device to a surface;
FIG. 9 is a side sectional view of a third embodiment of a securement assembly;
FIG. 10 is a side sectional view of the adhering element and securing element of the securement assembly of FIG. 9 shown securing a device to a surface;
FIG. 11 is a side sectional view of a fourth embodiment of a securement assembly;
FIG. 12 is a top view of the adhering element of FIG. 11;
FIG. 13 is a top view of a fifth embodiment of a securement assembly including an opening 350;
FIG. 14 is a side view of a sixth embodiment of a securement assembly.

While the above-identified drawings and figures set forth embodiments of the invention, other embodiments are also contemplated, as noted in the discussion. In all cases, this disclosure presents the invention by way of representation and not limitation. It should be understood that numerous other modifications and embodiments can be devised by those skilled in the art, which fall within the scope of this invention. The figures may not be drawn to scale.

### Detailed Description

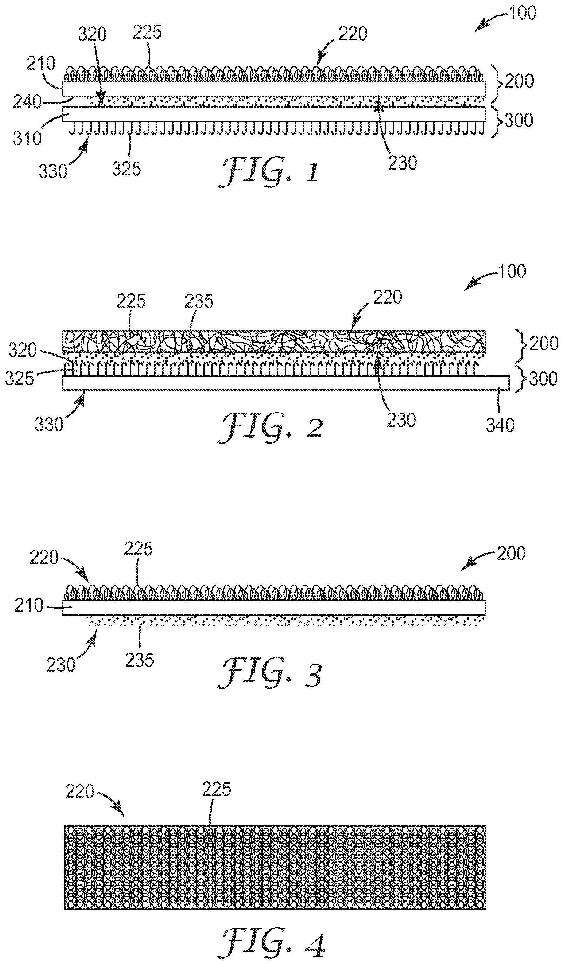

FIG. 1 is a side sectional view of a first embodiment of a securement assembly 100, which comprises an adhering element 200 and a securing element 300. The securing element 300 includes second mechanical fastener 325, discussed in detail below, which in this embodiment faces outward from the securing assembly 100. FIG. 2 is a side sectional view of a second embodiment of a securement assembly 100, which comprises an adhering element 200 and a securing element 300. This embodiment is similar to FIG. 1; however, in this embodiment, the securement element 300 includes second mechanical fastener 325 that faces the adhesive 235 of the adhering element 200. The similar elements of these two embodiments will be discussed in detail below.

FIG. 3 is a side sectional view of the adhering element 200 of FIG. 1. In this embodiment, the adhering element 200 includes a backing 210. FIG. 4 is a top view of the adhering element 200 of FIG. 3. The adhering element 200 comprises a first surface 220 that includes a first mechanical fastener 225 (described in more detail below) and a second surface 230, opposite the first surface 220, which includes adhesive 235. As shown in the embodiment of FIG. 3, an optional backing 210 is included, which may be one more layers of material to which the adhesive 235 is attached. As shown in FIG. 2, there is not an additional backing secured to the first mechanical fastener 225, and the first mechanical fastener 225 itself forms the first surface 220 and second surface 230 of the adhering element 200 to which the adhesive 235 is applied directly.

The adhesive 235 may be any type of adhesive useful for securing the adhering element 200 to a surface 500. The type of adhesive selected will depend on the desired properties of peel force, shear force, permanence, removability, characteristics of adherent, etc. The adhesive 235 will be a pressure sensitive adhesive, which can adhere to a surface with application of light pressure and without the need for heat or other external sources to active adhesion. For example, the adhesive may be an acrylate, silicone, urethane, polyolefin, synthetic rubber, or natural rubber based adhesive.

When the surface to which the adhering element is applied is skin, it may be desirable that the adhesive 235 hold well to the skin, but does not irritate or damage the skin during use or upon removal. Commonly used adhesives for application to skin are acrylate and silicone adhesives. Acrylate adhesives are well suited for securing to skin. The adhesion can be manipulated to have high adhesion or low adhesion. Suitable acrylate adhesives that can be applied to skin such as the acrylate copolymers are described in U.S. Patent No. RE 24,906. In particular, a 97:3 iso-octyl acrylate:acrylamide copolymer. Another acrylate adhesive is an 70:15:15 isooctyl acrylate: ethyleneoxide acrylate:acrylic acid terpolymer, as described in U.S. Pat. No. 4,737,410 (Example 31). Other useful acrylate adhesives are described in U.S. Pat. Nos. 3,389,827; 4,112,213; 4,310,509; and 4,323,557.

Generally, silicone adhesives are able to effectively secure dressings and tapes to skin and upon removal from the skin produce little or no skin damage. Typically, the silicone adhesives do not adhere well to polymer-based substrates, like tubing or hardgoods. The gentle removal of silicone adhesives from skin makes silicone adhesives well suited as the adhesive intended to contact the skin.

An example of a suitable silicone adhesive is disclosed in PCT Publications WO2010/056541 and WO2010/056543. A radiation cured silicone adhesive is particularly well suited for this application because the extent of crosslinking, and therefore adhesion of the silicone adhesive can be better controlled. Other examples of silicone gel adhesives systems include products marketed with the trade names: Dow Corning MG 7-9850, Wacker SILPURAN® 2110 and 2130, Bluestar SILBIONE® RT Gel 4317 and 4320, Nusil MED-6345 and 6350.

Optionally, backing 210 on the adhering element 200, if included, could be treated physically (e.g., corona, plasma, or flame treatment) or chemically (e.g., primer, adhesion promoter) before the adhesive 235 is applied in order to enhance the adhesion of adhesive 235 on backing 210.

The adhesive 235 may cover all or a portion of the second surface 230 of the adhering element 200. Also, the adhesive 235 may be applied to the second surface 230 in a discontinuous pattern or may be perforated. When the adhering element 200 is applied to skin, it is particularly desirable to have perforations or a pattern coating of adhesive to allow for moisture vapor to move away from the skin and out of the adhering element 200. For example, U.S. Pat. No. 4,595,001 and U.S. Pat. App. Pub. 2008-0233348, disclose a method of pattern coating adhesive.

The adhering element 200, and backing 210 if included, may be constructed from any number of common materials such as, for example, paper, film, foam, fabric, or nonwoven, to which the adhesive 235 is applied. The adhering element 200, and backing 210 if included, may comprise one or more layers of materials secured to one another. In some embodiments, the material selected for the adhering element 200 may itself serve as the first mechanical fastener 225, such as shown in FIG. 2. As shown in FIGS. 1 and 3, the adhering element 200 include a thin paper or film backing 210, with a loop filament on the surface of the backing 210. As shown in FIG. 2, the adhering element 200 includes a nonwoven material forming first mechanical fastener 225.

Overall, the thickness, stiffness, flexibility, permeability of the adhering element 200 will depend on the particular application and surface 500 to which the adhering element 200 is secured. For example, if the surface 500 is skin it may be desirable that the adhering element 200 is highly flexible and conformable to anatomical surface. Also, it may be desirable that the adhering element 200 allow for high moisture vapor permeability, but generally impermeable to liquid water so that microbes and other contaminants are sealed out from the area under the adhering element 200. One example of a suitable material is nonwoven or woven fabric. Another example of a suitable material is a high moisture vapor permeable film such as described in US Patents 3,645,835 and 4,595,001, the disclosures of which are herein incorporated by reference. In high moisture vapor permeable film/adhesive composites, the composite should transmit moisture vapor at a rate equal to or greater than human skin such as, for example, at a rate of at least 300 g/m² /24 hrs at 37°C/100-10% RH, or at least 700 g/m² /24 hrs at 37°C/100-10% RH, or at least 2000 g/m² /24 hrs at 37°C/100-10% RH using the inverted cup method as described in U.S. Pat. No. 4,595,001. In one embodiment, the backing 210 is an elastomeric polyurethane, polyester, or polyether block amide films. These films combine the desirable properties of resiliency, high moisture vapor permeability, and transparency.

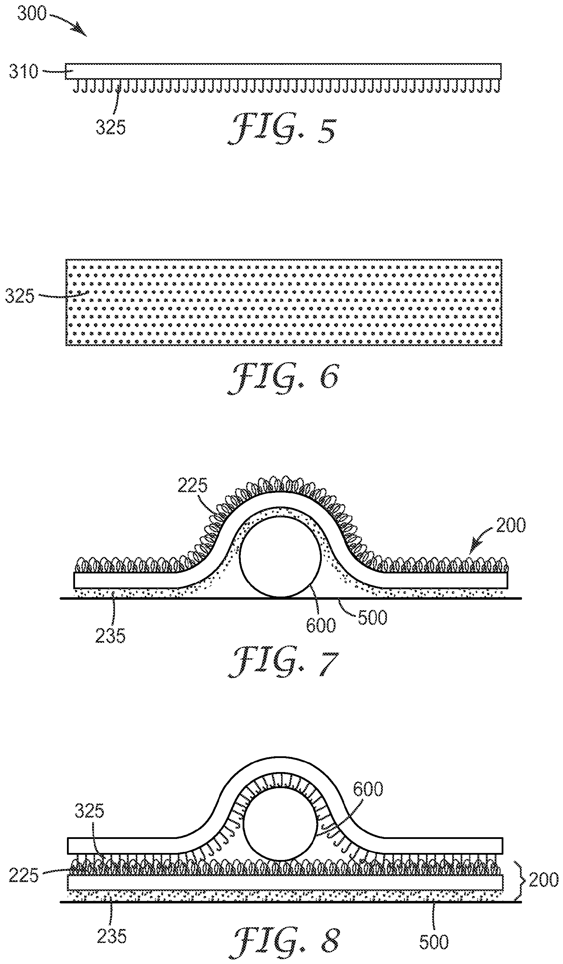

FIG. 5 is a side sectional view of the securing element 300, shown in either FIG. 1 or FIG. 2. FIG. 6 is a bottom view of the securing element 300 shown in FIG. 5. The securing element 300 comprises a first surface 320 and a second surface 330, opposite the first surface 320. When assembled with the adhering element 200, such as shown in FIGS. 1 or 2, the first surface 320 of the securing element 300 covers the adhesive 235 of the adhering element 200. In the embodiment shown in FIG. 1, the second surface 330 includes the second mechanical fastener 325. In the embodiment shown in FIG. 2, the first surface 320 includes the second mechanical fastener 325. As shown in FIG. 5, an optional backing 310 may be included as one more layers of material to form either the first surface 320 or second surface 330. The backing 310 is optional because the second mechanical fastener 325 may be formed to include both the fastener and a backing-like portion, all of the same material.

First mechanical fastener 225 and second mechanical fastener 325 are selected so that a secure mechanical, interlocking interaction is formed when placed in contact with one another. In one embodiment, the first mechanical fastener 225 and second mechanical fastener 325 are reclosable, so that an interlocking interaction is formed that can be opened and reclosed. In one embodiment, the first mechanical fastener 225 and second mechanical fastener 325 are able to interlock with one another to secure, but with sufficient force are able to disengage. In one embodiment, depending on the selection of both materials for achieving mechanical fastening, the first or second mechanical fasteners may comprise various constructions of hooks, loops, or stems, each of which may include selectively placed adhesive.

In one embodiment, as shown in FIGS. 1-3, the first mechanical fastener 225 comprises loop material and the second mechanical fastener 325 comprises a plurality of hooks. Loop material can be any suitable material that interlocks with a corresponding hook. In some embodiment, the loop material includes one or more filaments or fibers that may be arranged as knitted, woven, or non-woven fabrics. The term "non-woven" refers to a material having a structure of individual fibers or threads that are interlaid but not in an identifiable manner such as in a knitted fabric. Examples of non-woven webs include spunbond webs, spunlaced webs, airlaid webs, meltblown web, and bonded carded webs. In some embodiment, the loop material itself comprises the adhering element 200, with no additional backing 210. In some embodiments, the loop material comprises a fibrous layer disposed on the backing 210. Exemplary suitable loop materials are described, for example, in U.S. Pat. Nos. 5,616,394; 5,256,231; and 5,389,416. As described in U.S. Pat. No. 5,256,231, the fibrous layer in a loop material according to some embodiments disclosed herein comprises arcuate portions projecting in the same direction from spaced anchor portions on the backing.

A hook as used herein is a fastening element that interlocks with a corresponding hook or interlocks with a loop material. Typically, a plurality of hooks are provided. In some embodiment, a hook is a generally outward projecting post with a laterally extending portion, which can interlock with a corresponding hook or loop material. Exemplary hook designs are disclosed in U.S. Pat. Nos. 5,845,375; 5,077,870; 5,607,635; and 5,679,302.

The hooks can be integral (that is, formed at the same time as a unit, unitary) with a backing. Upstanding posts on a backing can be made, for example, by feeding a thermoplastic material onto a continuously moving mold surface with cavities having the inverse shape of the posts. The thermoplastic material can be passed between a nip formed by two rolls or a nip between a die face and roll surface, with at least one of the rolls having the cavities. Pressure provided by the nip forces the resin into the cavities. In some embodiments, a vacuum can be used to evacuate the cavities for easier filling of the cavities. The nip typically has a large enough gap such that a coherent backing is formed over the cavities. The mold surface and cavities can optionally be air or water cooled before stripping the integrally formed backing and upstanding hook elements from the mold surface such as by a stripper roll. If the posts formed upon exiting the cavities do not have loop-engaging heads, loop-engaging heads could be subsequently formed into hooks by a capping method as described in U.S. Pat. No. 5,077,870 (Melbye et al.). Typically, the capping method includes deforming the tip portions of the hook elements using heat and/or pressure. The heat and pressure, if both are used, could be applied sequentially or simultaneously.

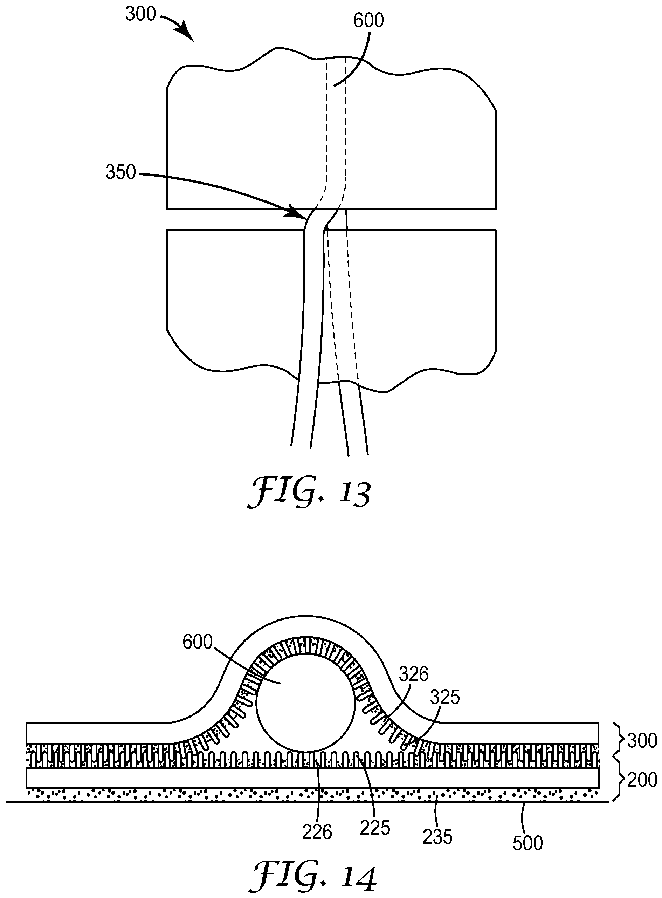

A stem as used herein is a fastening element that could be used as the first and/or second mechanical fastener. A stem is a protrusion that projects from a surface. While hooks generally have an overhang, or other portion that projects laterally from the main body of the hook to create the interlocking fastening system, a stem typically does not have a portion that projects laterally from the main body of the stem. In one embodiment, a stem will have linear side walls or slightly tapered side walls. A plurality of stems provide for a high amount of surface area to resist shear forces between the stems and the surface it is contacting. Fig. 14 below shows and describes examples of how stems can be incorporated as one of the first and/or second mechanical fasteners.

In one embodiment, the securing element 300 covers the adhesive 235 of the adhering element 200 to serve as a protective liner over the adhesive 235 of the adhering element 200. The securing element 300 is removable from the adhesive 235 of the adhering element 200. Specifically, the first surface 320 of the securing element 300 can be removed from the adhesive 235. In one embodiment, once the securing element 300 is removed, the adhering element 200 (FIG. 3) and securing element 300 (FIG. 5) separate from one another. It is understood that, the adhering element 200 and securing element 300 do not necessarily need to be entirely disconnected from one another and in some embodiments may be partially connected to one another. In one embodiment, the adhesive properties can be manipulated to aid in the removal of the securing element 300 from the adhering element 200. In one embodiment, the first surface 320 of the securing element 300 may include a coating, such as a low adhesion backsize (LAB) or a silicone release coating to aid in the removal of the securing element 300 from the adhering element 200. In another embodiment, such as shown in FIG. 2, the second mechanical fastener 325 itself may aid in removal of the securing element 300 from the adhering element 200. For example, when the second mechanical fastener 325 is a plurality of hooks, the hooks provided less surface area contact with the adhesive 235 and therefore limit the force required to remove the securing element 300 from the adhering element 200. Optionally, the second mechanical fastener 325 can be surface coated with an adhesion modifier coating.

In one embodiment, a portion of the securing element 300 extends beyond the adhesive 235 of the adhering element 200 to form a tab 340 to aid in removing the securing element 300 from the adhering element 200. In one embodiment, the adhesive 235 may not extend completely to the edge of the second surface 230 of the adhering element 200 such that a tab 240 is formed to aid in removing the securing element 300 from the adhering element 200. In one embodiment, such as would be applicable for FIG. 2, a portion of the second mechanical fastener 325, if hooks or stems, can be crushed such as described in U.S. Patent 5,933,927, the disclosure of which is herein incorporated by reference. When crushing a portion of the second mechanical fasteners 325, the recessed portion from the crushed hooks or stems does not sufficiently contact the adhesive 235 and therefore forms a tab 240.

The securing element 300 can be formed of one or more layers of material, such as, for example, paper, film, foam, fabric, or nonwoven. Typically, the securing element 300 will be relatively flexible to allow for a drapable arrangement over a device 600 while still coming into contact with the first mechanical fastener 225 on the adhering element 200 (see FIGS. 8, 10, 11).

The exposed adhesive 235 of the adhering element 200 can be applied to a surface 500. In one embodiment, the adhering element 200 can be used alone for securement to a surface 500. For example, FIG. 7 shows a side sectional view of the adhering element 200 of the securement assembly 100 of FIG. 1 or FIG. 2 shown securing a device 600 to a surface 500. As can be seen, the device 600 is placed in contact with the surface 500 while the adhering element 200 overlies the device 600 and secures the device to the surface 500. Extending outward from the adhering element 200 is the first mechanical fastener 225.

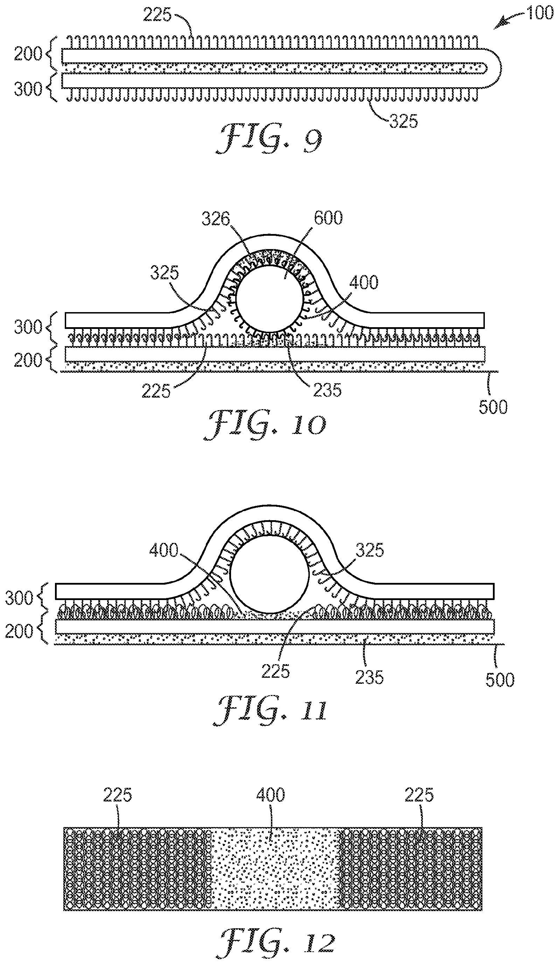

In one embodiment, the adhering element 200 can be used with the securing element 300 for securement with an optional underlying device 600 to a surface 500. For example, FIG. 8 (similarly FIGS. 10, 11, and 14) shows a side sectional view of the adhering element 200 and securing element 300 of the securement assembly 100 of FIG. 1 or FIG. 2 shown securing a device 600 to a surface 500. As can be seen, the adhering element 200 is fully applied in contact with the surface 500. A device 600 is positioned over a portion of the first surface 220. The second mechanical fastener 325 of the securing element 300 is applied over the first surface 220 of the adhering element 200, containing the first mechanical fastener 225. The first mechanical fastener 225 and second mechanical fastener 325 securely connect to one another to secure the device 600. In this embodiment, the device 600 is supported by the adhering element 200 and is not in direct contact with the surface 500. Therefore, the adhering element 200 serves to protect the underlying surface 500 from contact with the device 600.

The securement assembly 100 shown in FIGS. 9 and 10 is similar to the previously disclosed securement assembly 100 described in FIGS. 1-8. However, in the embodiments in FIGS. 9 and 10, the first mechanical fastener 225 comprises a plurality of hooks and the second mechanical fastener 325 comprises a plurality of hooks. Various designs of interlocking hook/hook designs
as those disclosed in U.S. Pat. 5,607,636.

In addition, FIG. 9 shows that a portion of the adhering element 200 can be integrally connected with a portion of the securing element 300, such that the two do not entirely separate from one another. However, the securing element 300 can move from a position of covering the adhesive 235 of the adhering element 200, as shown in FIG. 9, to a position wherein the first mechanical fastener 225 and second mechanical fastener 325 contact one another.

In addition, FIG. 10 shows an optional friction control element 400. A friction control element 400 is used to further secure the device 600 to the securement assembly 100. The arrangement such as shown in FIG. 8 would provide for securement of the underlying device 600 from a force pulling up on the device. However, there may be little resistance to a laterally applied force. A friction control element 400 can be included to provide securement to a laterally applied force. It is understood that in some embodiments, the construction of the adhering element and/or the securing element itself may provide some resistance to a laterally applied force.

As shown in FIG. 10, the friction control element 400 is directly on the device 600. In this embodiment, the friction control element 400 is a plurality of hooks on the device 600 that secure with both the first mechanical fastener 225 and second mechanical fastener 325. These hooks on the device 600 could be provided as a material applied to the device or created directly on the device.

It is understood that any suitable material can be provided that when in contact with the device 600 can provide additional securement. For example, stems (see US Patents 6,372,323; 6,610,382; and 6,904,615), hook material, loop material, or adhesive could be used to further secure the device 600. The friction control element 400 can be located on the device 600, such as shown in FIG. 10. In other embodiments the friction control element 400 can be located on one or both of the adhering element 200 or securing element 300. It is understood that the friction control element 400 may be provided on only a portion of one or both of the adhering element 200, securing element 300, or device 600. For example, FIG. 11 and FIG. 12 show another embodiment of a securement assembly 100 with a construction similar to that shown and described in FIGS. 1-8. However, in this embodiment, the securing element 300 includes a friction control element 400 on a portion of the first surface 220 of the adhering element 200. As can be seen in FIG. 12, the friction control element 400 is in a center portion of the adhering element 200 with the first mechanical fastener 225 on either side. In this embodiment, the friction control element 400 is adhesive.

Optionally, the first mechanical fastener 225, second mechanical fastener 325 or both may include a working adhesive 226, 326, respectively, such as shown in FIGS. 10 and 14. This working adhesive 226, 326 can further aid in securing directly with the device 600 such as shown in FIG. 10. Also, the working adhesive 226, 326 can further provide securement of the first mechanical fastener 225 to the second mechanical fastener 325, shown more specifically in FIG. 14. In one embodiment, the working adhesive 226 is recessed from the outermost portion of the first mechanical fastener 225. In one embodiment, the working adhesive 326 is recessed from the outermost portion of the second mechanical fastener 325. In one embodiment, the working adhesive 226 and 326 is recessed from both the first and second mechanical fasteners 325, as shown in FIG. 14. The extent to which the mechanical fasteners are arranged and the extent of the recess of the recessed adhesive can impact whether or not that surface will feel tacky to a user's touch. In one embodiment, either the first surface 220 having the first mechanical fastener 225 and recessed working adhesive, or the surface having the second mechanical fastener 325 and recessed working adhesive are not sticky to the user's touch. When the first mechanical fastener 225 and second mechanical fastener 325 are stems, such as shown in FIG. 14, or hooks, the protruding portions of the stem or hook can extend into the space containing the adhesive 226, 326, even if only one of the surfaces includes adhesive, and high levels of securement are achieved.

Specifically, FIG. 14 shows a securement assembly 100 with an adhering element 200 and a securing element 300. On the first surface 220 of the adhering element 200 is the first mechanical fastener 225, which comprises a plurality of stems. Also on the first surface 220 is the first working adhesive 226 that is recessed from the outermost portion of the stems. The securing element 300 would initially cover the adhesive 235 of the adhering element 200 similar to that shown in either FIGS. 1 or 2. However, FIG. 14 shows the securing element 330 and the second mechanical fastener 325 applied over the first mechanical fastener 225 of the adhering element 200. In this embodiment, the second mechanical fastener 325 also comprises a plurality of stems and also includes a second working adhesive 326 that is recessed from the outermost portion of the stems. During use the stems interact with one another to provide mechanical securement, specifically from applied shear forces, while the first and second working adhesive 226, 326 interact to resist peel forces. Examples of stems that would be useful as first or second mechanical fastener are described in US Pat. No. 4,959,265; 6,372,323; 6,610,382; and 6,904,615.

It is understood that only one of the first mechanical fastener 225 or second mechanical fastener 325 could include a working adhesive, while still achieving high resistance to shear forces and peel forces, because the stems of the overlying sheet will embed into the working adhesive.

In one embodiment, the stems are included in a density of at least 500 stems per square inch. In one embodiment, the stems are included in a density of at least 1000 stems per square inch. In one embodiment the stems have a height at least 50 microns. In one embodiment the stems have a height of at least 100 microns. In one embodiment, the stems have a height less than 500 microns.

When a working adhesive is included in an embodiment where the mechanical fastener is either stems or hooks, the working adhesive may be provided the full height of the stems or hooks or may be recessed from the stems or hooks. Such recessed working adhesives on a plurality of stems or hooks may be coated in a variety of ways, including solvent coating or bulk coating.

A variety of factors, such a stem/hook density, stem/hook flexibility may impact the apparent tackiness of the recessed adhesive. High tackiness may help enable securement of the secured tubing or medical device, but low tackiness prevents the material from sticking to gloved fingers. In one embodiment, a plurality of stems/hooks with a working adhesive will have not finger tack with the working adhesive provided less that 25% of the stem/hook height. In one embodiment, a plurality of stems/hooks with a working adhesive will have slight finger tack with the working adhesive provided between 25% and 45% of the stem/hook height. In one embodiment, a plurality of stems/hooks with a working adhesive will have good finger tack with the working adhesive provided at greater than 45% of the stem/hook height. It is understood that a protective release liner could be used to cover the surface that contains a working adhesive.

Optionally, securing element 300 could have an opening 350, wherein at least a portion of the device 600 could pass through. FIG. 13 shows an example of a securing element 300 with a device 600 passing though from the first surface 320 to the second surface 330. It is understood that the opening 350 may include a throughhole that the device 600 passes through, a slit that wraps around the device 600, or as shown in FIG. 13, two parts of securing element 300 that wrap around the device 600. It is understood that although not specifically shown in the view, an underlying adhering element 200 is included for the securement assembly 100 to secure with the surface 500 and to which the shown securing element 300 is attached. The surface is skin and the device is a medical device, such as a tube, catheter, port, or pump.

The securement assembly 100 can be provided in any number of forms. For example, the securement assembly 100 may be an elongated in a roll and torn or cut to the desired length for use. However, for the construction shown and described in FIG. 1 and 9, roll form may be less desirable as the outward projecting hook will interlock with the underlying/overlying hook or loop of the roll. Alternatively, precut strips, sections, in various sizes and shapes, which include medical dressings, may be used to provide the securement assembly 100. Medical dressings are used for a number of applications, such as, but not limited to catheter securement or wound coverings. Depending on the application, the securement assembly 100 may be provided in a sterile package.

Although the first and second mechanical fastener 225, 325 are shown over most of the surface of the adhering element 200 and securing element 300, respectively, it is understood that either adhering element 200 or securing element 300 may include the mechanical fasteners over only a small portion of the surface area. For example, a medical dressing, such as a dressing used to cover an intravenous catheter may use the securement assembly 100 construction such that the adhering element 200 is applied to skin and the overlying securing element 300 covers and secures the outwardly projecting medical tubes. In this example, only portions of either the adhering element 200 or securing element 300 might include the mechanical fasteners.

Various embodiments have been described. It is understood that the medical dressing can be provided in a number of sizes, shapes, configurations, in a package, sterilized, with or without liners covering any of the exposed or tacky adhesive surfaces. It is understood that various features, material, components described in one of the embodiment could be used and incorporated into one or more of the other disclosed embodiments. For example any one of the described adhering element could be used with any one of the described securing elements. Additionally, it is understood that additional features such as working adhesive, tabs, or backings described with one embodiment may be applicable to other embodiments.

Although specific embodiments of this invention have been shown and described herein, it is understood that these embodiments are merely illustrative of the many possible specific arrangements that can be devised in application of the principles of the invention. Numerous and varied other arrangements can be devised in accordance with these principles by those of ordinary skill in the art without departing from scope of the invention. Thus, the scope of the present invention should not be limited to the structures described in this application, but only by the structures described by the language of the claims and the equivalents of those structures.

## Claims

1. A securement assembly (100) for securing a medical device (600) to a skin surface, the securement assembly comprising:
an adhering element (200) comprising:
a first surface (220) comprising a first mechanical fastener (225);
a second surface (230), opposite the first surface (220), comprising an adhesive (235) for securing the adhering element (200) to the surface;
a securing element (300) comprising:
a first surface (320);
a second surface (330), opposite the first surface (320);
a second mechanical fastener (325) on at least the first surface (320) or second surface (330) of the securing element (300), wherein the second mechanical
fastener (325) is configured to secure with the first mechanical fastener (225);
wherein the first surface (320) of the securing element (300) covers and releasably secures to the adhesive (235) of the second surface (230) of the adhering element (200) and wherein the adhesive (235) is a pressure-sensitive adhesive.

2. The securement assembly (100) of claim 1, wherein the pressure-sensitive adhesive is an acrylate, a silicone, a urethane, a polyolefin, a synthetic rubber or a natural rubber based adhesive.

3. The securement assembly (100) of any one of the preceding claims, wherein the first mechanical fastener (225) comprises a plurality of hooks, loops, or stems.

4. The securement assembly (100) of any one of the preceding claims, wherein a portion of
the securing element (300) extends beyond the second surface (230) of the adhering element (200) forming a tab (340).

5. The securement assembly (100) of any one of the preceding claims, wherein the second mechanical fastener (325) is on the first surface (320) of the securing element (300).

6. The securement assembly (100) of any one of the preceding claims, wherein the second mechanical fastener (325) is on the second surface (330) of the securing element (300).

7. The securement assembly (100) of any one of claims 1-4, wherein the second mechanical fastener (325) is on the second surface (330) of the securing element (300) and the first surface (320) of the securing element (300) includes a release coating.

8. The securement assembly (100) of any one of the preceding claims, further comprising a friction control element (400) placed on at least one of the adhering element (200) or securing element (300) for securement to the medical device (600).

9. The securement assembly (100) of any one of claims 1-7, further comprising a friction control element (400) that is a third mechanical fastener on the medical device (600) configured to secure with at least one of the first mechanical fastener (225) or second mechanical fastener (325).

10. The securement assembly (100) of any one of the preceding claims, further comprising a working adhesive embedded in at least one of the first mechanical fastener (225) or second mechanical fastener (325).

11. The securement assembly (100) of claim 1, wherein the second mechanical fastener (325) secures to the first mechanical fastener (225); and
wherein the medical device (600) is positioned between a portion of the first surface (220) of the adhering element (200) containing the first mechanical fastener (225) and the overlying second mechanical fastener (325).

## Patentansprüche

1. Befestigungsanordnung (100) zum Sichern einer medizinischen Vorrichtung (600) an einer Hautoberfläche, wobei die Befestigungsanordnung Folgendes aufweist:
ein haftendes Element (200), aufweisend:
eine erste Oberfläche (220), die ein erstes mechanisches Befestigungsmittel (225) aufweist;
eine zweite Oberfläche (230), die der ersten Oberfläche (220) entgegengesetzt ist, und die einen Kleber (235) zum Sichern des haftenden Elements (200) an der Oberfläche aufweist;
ein Sicherungselement (300), aufweisend:
eine erste Oberfläche (320);
eine zweite Oberfläche (330), die der ersten Oberfläche (320) entgegengesetzt ist;
ein zweites mechanisches Befestigungsmittel (325) auf mindestens der ersten Oberfläche (320) oder der zweiten Oberfläche (330) des Sicherungselements (300), wobei das zweite mechanische Befestigungsmittel (325) für eine Sicherung mit dem ersten mechanischen Befestigungsmittel (225) ausgelegt ist;
wobei die erste Oberfläche (320) des Sicherungselements (300) den Kleber (235) der zweiten Oberfläche (230) des haftenden Elements (200) bedeckt und lösbar daran festgehalten wird, und wobei der Kleber (235) ein Haftkleber ist.

2. Befestigungsanordnung (100) nach Anspruch 1, wobei der Haftkleber ein Kleber auf Basis von Acrylat, Silikon, Urethan, Polyolefin, synthetischem Kautschuk oder Naturkautschuk ist.

3. Befestigungsanordnung (100) nach einem der vorstehenden Ansprüche, wobei das erste mechanische Befestigungsmittel (225) mehrere Haken, Schlaufen oder Stiele aufweist.

4. Befestigungsanordnung (100) nach einem der vorstehenden Ansprüche, wobei ein Abschnitt des Sicherungselements (300) über die zweite Oberfläche (230) des haftenden Elements (200) vorsteht und eine Lasche (340) bildet.

5. Befestigungsanordnung (100) nach einem der vorstehenden Ansprüche, wobei sich das zweite mechanische Befestigungsmittel (325) auf der ersten Oberfläche (320) des Sicherungselements (300) befindet.

6. Befestigungsanordnung (100) nach einem der vorstehenden Ansprüche, wobei sich das zweite mechanische Befestigungsmittel (325) auf der zweiten Oberfläche (330) des Sicherungselements (300) befindet.

7. Befestigungsanordnung (100) nach einem der Ansprüche 1 bis 4, wobei sich das zweite mechanische Befestigungsmittel (325) auf der zweiten Oberfläche (330) des Sicherungselements (300) befindet und die erste Oberfläche (320) des Sicherungselements (300) eine Trennbeschichtung einschließt.

8. Befestigungsanordnung (100) nach einem der vorstehenden Ansprüche, ferner ein Reibungsbegrenzungselement (400) aufweisend, das auf mindestens einem von dem haftenden Element (200) oder dem Sicherungselement (300) zur Sicherung an der medizinischen Vorrichtung (600) angeordnet ist.

9. Befestigungsanordnung (100) nach einem der Ansprüche 1 bis 7, ferner ein Reibungsbegrenzungselement (400) aufweisend, das ein drittes mechanisches Befestigungsmittel auf der medizinischen Vorrichtung (600) ist, das zur Sicherung mit mindestens einem von dem ersten mechanischen Befestigungsmittel (225) oder dem zweiten mechanischen Befestigungsmittel (325) ausgelegt ist.

10. Befestigungsanordnung (100) nach einem der vorstehenden Ansprüche, ferner einen Funktionskleber aufweisend, der in mindestens eines von dem ersten mechanischen Befestigungsmittel (225) oder dem zweiten mechanischen Befestigungsmittel (325) eingebettet ist.

11. Befestigungsanordnung (100) nach Anspruch 1, wobei das zweite mechanische Befestigungsmittel (325) am ersten mechanischen Befestigungsmittel (225) gesichert wird; und
wobei die medizinische Vorrichtung (600) zwischen einem Abschnitt der ersten Oberfläche (220) des haftenden Elements (200), welches das erste mechanische Befestigungsmittel (225) aufweist, und dem darüberliegenden zweiten medizinischen Befestigungsmittel (325) angeordnet ist.

## Revendications

1. Ensemble de fixation (100) pour fixer un dispositif médical (600) à une surface de la peau,
l'ensemble de fixation comprenant :
un élément adhérent (200) comprenant :
une première surface (220) comprenant un premier élément d'attache mécanique (225) ;
une deuxième surface (230), opposée à la première surface (220), comprenant un adhésif (235) pour fixer l'élément adhérent (200) à la surface ;
un élément de fixation (300) comprenant :
une première surface (320) ;
une deuxième surface (330), opposée à la première surface (320) ;
un deuxième élément d'attache mécanique (325) sur au moins la première surface (320) ou la deuxième surface (330) de l'élément de fixation (300), dans lequel le deuxième élément d'attache mécanique (325) est configuré pour se fixer au premier élément d'attache mécanique (225) ;
dans lequel la première surface (320) de l'élément de fixation (300) couvre, et se fixe de manière amovible à, l'adhésif (235) de la deuxième surface (230) de l'élément adhérent (200) et dans lequel l'adhésif (235) est un adhésif sensible à la pression.

2. Ensemble de fixation (100) selon la revendication 1, dans lequel l'adhésif sensible à la pression est un adhésif à base d'acrylate, de silicone, d'uréthane, de polyoléfine, de caoutchouc synthétique ou de caoutchouc naturel.

3. Ensemble de fixation (100) selon l'une quelconque des revendications précédentes, dans lequel le premier élément d'attache mécanique (225) comprend une pluralité de crochets, boucles ou tiges.

4. Ensemble de fixation (100) selon l'une quelconque des revendications précédentes, dans lequel une partie de
l'élément de fixation (300) s'étend au-delà de la deuxième surface (230) de l'élément adhérent (200) en formant une languette (340).

5. Ensemble de fixation (100) selon l'une quelconque des revendications précédentes, dans lequel le deuxième élément d'attache mécanique (325) est sur la première surface (320) de l'élément de fixation (300).

6. Ensemble de fixation (100) selon l'une quelconque des revendications précédentes, dans lequel le deuxième élément d'attache mécanique (325) est sur la deuxième surface (330) de l'élément de fixation (300).

7. Ensemble de fixation (100) selon l'une quelconque des revendications 1 à 4, dans lequel le deuxième élément d'attache mécanique (325) est sur la deuxième surface (330) de l'élément de fixation (300) et la première surface (320) de l'élément de fixation (300) inclut un revêtement de libération.

8. Ensemble de fixation (100) selon l'une quelconque des revendications précédentes, comprenant en outre un élément de régulation de frottement (400) placé sur au moins l'un parmi l'élément adhérent (200) ou l'élément de fixation (300) pour fixation au dispositif médical (600).

9. Ensemble de fixation (100) selon l'une quelconque des revendications 1 à 7, comprenant en outre un élément de régulation de frottement (400) qui est un troisième élément d'attache mécanique sur le dispositif médical (600) configuré pour se fixer à au moins l'un parmi le premier élément d'attache mécanique (225) ou le deuxième élément d'attache mécanique (325).

10. Ensemble de fixation (100) selon l'une quelconque des revendications précédentes, comprenant en outre un adhésif de travail intégré dans au moins l'un parmi le premier élément d'attache mécanique (225) ou le deuxième élément d'attache mécanique (325).

11. Ensemble de fixation (100) selon la revendication 1, dans lequel le deuxième élément d'attache mécanique (325) se fixe au premier élément d'attache mécanique (225) ; et
dans lequel le dispositif médical (600) est positionné entre une partie de la première surface (220) de l'élément adhérent (200) contenant le premier élément d'attache mécanique (225) et le deuxième élément d'attache mécanique (325) qui se trouve au-dessus.
